# EUROPEAN PATENT SPECIFICATION

(11) **EP 2 733 202 B1**
(45) Date of publication and mention of the grant of the patent: **22.08.2018**
(21) Application number: 13001847.6
(22) Date of filing: 10.04.2013
(51) Int. Cl.: C12N 5/071, C12N 5/0797, C12N 15/867, C12N 15/861, C12N 15/85

(54) **The INS cells and the method for reprogramming somatic cells to INS cells using Sox2 and c-Myc**
INS-Zellen sowie Verfahren zur Umprogrammierung somatischer Zellen in INS-Zellen unter Verwendung von Sox2 und c-Myc
Cellules INS et procédé de reprogrammation de cellules somatiques sur des cellules INS à l'aide de Sox2 et c-Myc

(30) Priority: 15.11.2012 PL 40163312
(43) Date of publication of application: 21.05.2014
(73) Proprietor: Celther Polska Sp. z o.o., 05-170 Zakroczym (PL)
(72) Inventor: Rieske, Piotr, 91-013 Lódz (PL); Stoczynska-Fidelus, Ewelina, 96-124 Maków (PL); Piaskowski, Sylwester, 95-050 Konstantynów (PL); Grzela, Dawid, 25-453 Kielce (PL)
(74) Representative: Kondrat, Mariusz

(56) References cited:
- WO-A2-2012/022725
- WO-A2-2012/174467
- US-A1- 2012 269 778
- KARENL RING ET AL: "Direct Reprogramming of Mouse and Human Fibroblasts into Multipotent Neural Stem Cells with a Single Factor", CELL STEM CELL, CELL PRESS, US, vol. 11, no. 1, 15 May 2012 (2012-05-15), pages 100-109, XP028400409, ISSN: 1934-5909, DOI: 10.1016/J.STEM.2012.05.018 [retrieved on 2012-05-16]
- GIORGETTI ALESSANDRA ET AL: "Cord blood-derived neuronal cells by ectopic expression of Sox2 and c-Myc", PROCEEDINGS OF THE NATIONAL ACADEMY OF SCIENCES OF THE UNITED STATES OF AMERICA, vol. 109, no. 31, July 2012 (2012-07), pages 12556-12561, XP009171502, ISSN: 0027-8424
- ERNESTO LUJAN ET AL: "Direct conversion of mouse fibroblasts to self-renewing, tripotent neural precursor cells", PROCEEDINGS OF THE NATIONAL ACADEMY OF SCIENCES, NATIONAL ACADEMY OF SCIENCES, US, vol. 109, no. 7, 14 February 2012 (2012-02-14), pages 2527-2532, XP002679170, ISSN: 0027-8424, DOI: 10.1073/PNAS.1121003109
- DONG WOOK HAN ET AL: "Direct Reprogramming of Fibroblasts into Neural Stem Cells by Defined Factors", CELL STEM CELL, vol. 10, no. 4, 1 April 2012 (2012-04-01), pages 465-472, XP055027756, ISSN: 1934-5909, DOI: 10.1016/j.stem.2012.02.021

## Description

The subject of the present invention are the iNS (induced neural stem) cells that are characterized by the presence of the markers Sox2, nestin (neural stem cell markers) and the GFAP (glial fibrillary acidic protein) and the generation of iNS cells by reprogramming factor, and also the procedure for generation of derivatives characterized by the markers MAP2+ and beta tubulin III+ (neuronal cell markers) in these cells.

The subject of the present invention is also the method for reprogramming somatic cells (e.g. fibroblasts, keratinocytes) to iNS cells with reprogramming factors, Sox2 and c-Myc.

The present invention describes a reprogramming of somatic cells. Unlike the methods used so far, the cell dedifferentiation will be not performed to the iPS (induced pluripotent stem cells) stage, but to the stage of iNS cells (neural stem cells) that are characterized by the presence of markers (Sox2, GFAP and nestin). This method is more expedient because the risk for neoplastic transformation is lower and the differentiation of iNS cells into neural cells is easier than that of iPS cells. Focusing on the obtainment of cells, which have profiles more closely resembling the target ones, allows achievement of a higher efficiency of the reprogramming process and also of the differentiation by means of differentiation factors (e.g. bFGF) into cells characterized by the presence of the markers MAP2+ i beta III+. Barriers have been overcome and this created the possibility of obtaining a definite kind of multipotent cells, but not pluripotent cells. Multipotent cells have not the ability to cause teratomas. So far, SOX2 and MYC were used in combination with KLF4 and OCT4, however, in a set, which accomplishes a totally different objective than that proposed in the disclosure. So far, nobody has noticed that removing two of them allows obtainment of a definite kind of multipotent cells.

For many years scientists investigated the possibility of regeneration or reconstruction of damaged cells and tissues. For example, stem cells derived from embryos (ES) provide such possibilities, which result in enormous problems in respect of ethics and morality.

Such difficulty is not associated with the use of induced pluripotent stem cells (iPS).

Unlike the ES cells, iPS cells are not derived from embryos, but from somatic cells reprogrammed (regressed to a pluripotency state when these cells have the ability to differentiate into different kinds of cells) using specific techniques. First procedures of this kind were performed in 2007 in Japan [Takahashi K., Tanabe K., Ohnuki M., et al.: Induction of pluripotent stem cells from adult human fibroblasts by defined factors. Cell 2007; 131: 861-72.]. These scientists used a gene set (Oct4, Sox2, Klf4 and c-Myc - OSKM set), which was delivered into a somatic cell genome during a viral transduction. A similar experiment was performed in the Thompson Laboratory, but in this case a different set of genes, OCT4, Sox2, NANOG and LIN28 (OSNL) was used [Yu J., Vodyanik M.A., Smuga-Otto K. et al.: Induced pluripotent stem cell lines derived from human somatic cells; Science 2007; 318: 1971-20.]. During subsequent years, this technique was developed and attempts were made to increase the reaction efficiency and also to reduce the risk for neoplastic transformation of cells induced with this method (initially, dangerous c-Myc oncogenes were applied) [Nakagawa M., Koyanagi M., Tanabe K. et al.: Generation of induced pluripotent stem cells without Myc from mouse and human fibroblast. Nat Biotechnol 2008; 26: 101-6; Wernig M., Meissner A., Cassady J.P. et al.: c-Myc is dispensable for direct reprogramming of mouse fibroblasts. Stem Cell 2008; 2: 10-12.].

However, the removal of the c-Myc gene from the used gene set resulted in a considerable, 100-fold decrease in process efficiency, whereas the use of valproic acid, a histone deacetylase inhibitor, and also of a methyltransferase inhibitor again afforded possibilities for increasing its efficiency. This allowed also a reduction of the number of sites used for reprogramming factors to OCT4 and Sox2 [Huangfu K., Maehr R., Guo W. et al.: Induction of pluripotent stem cells by defined factors is greatly improved by small-molecule compounds. Nat Biotechnol 2008; 26: 795-7.].

Despite the elimination of hazardous factors, the risk for neoplastic transformation is high, because during retroviral transductions the introduced genes become integrated into the genome not accidentally, thus they can activate or inactivate genes that are of importance for normal cell function, which may bring about cell function disturbances and in consequence a neoplastic transformation. The risk for neoplastic transformation results from the fact that vectors sharing elements with the host's genome may induce insertional mutagenesis.

In order to reduce the risk of this phenomenon, it was attempted to use casettes containing all the factors necessary for a reprogramming. This afforded possibilities for decreasing the number of sites where viral vectors interfered with the cell genome [Sommer C., Stadtfeld M., Murphy G., Hochedlinger K., Kotton D.N., Mostoslavsky G.: Induced pluripotent stem cell generation using a single lentiviral stem cell cassette. Stem Cell 2009; 27: 543-9. Carey B.W., Markoulaki S., Hanna J., Saha K., Gao Q., Mitalipova M.: Reprogramming of murine and human somatic cells using a single polycistronic vector. Proc Natl Acad Sci USA 2009; 106: 157-62.].

Adenoviral vectors (not integrated into the host genome) [Stadtfeld M., Nagaya M., Utikal J., Weir G., Hochedlinger K.: Induced pluripotent stem cells generated without viral integration. Science 2008; 322: 945-9.], lentiviral vectors that became inactivated two weeks after their introduction into the cells (the time needed for reprogramming) [Maherali N., Ahfeldt T., Rigamonti A., Utikal J., Cowan C., Hochedlinger K.: High-efficiency system for the generation and study of human induced pluripotent stem cells. Cell Stem Cell 2008; 3: 340-5.] or a plasmid DNA transfection [Okita K., Nakagawa M., Hyenjong H., Ichisaka T., Yamanaka S.: Generation of mouse induced pluripotent stem cells without viral vectors. Science 2008; 322: 949-53.] were used to eliminate the risk of damage to host's genetic material completely.

Recent experiments focus on reprogramming with methods that eliminate the necessity of applying DNA molecules. Extracts containing proteins of the described above transcription factors as well as small-molecule chemical compounds (valproic acid, BIX-01294 and TGF-beta inhibitor) are used [Shi Y., Do J.T., Desponts C., Hahm H.S., Scholer H.R., Ding S.: A combined chemical and genetic approach for the generation of induced pluripotent stem cells. Cell Stem Cell 2008; 2: 525-8. Ichida J.K., Blanchard J., Lam K. et al. A small-molecule inhibitor of Tgf-beta signaling replaces Sox2 in reprogramming. Cell Stem Cell 2009; 5: 491-503.].

The transposons, autonomic genetic elements capable of moving within the cell genome in a process of non-homologous recombination called transposition, constitute a different system, which provides the possibility of safe use. Transposons are classified into two groups: (1) retrotransposons that transpose through an RNA intermediate, and (2) DNA transposons that are characterized by movement of a DNA segment limited by terminal repeats of a transposon through the genome.

In the process of translocation of the second group elements, transposons are cut out from one site (donor locus - genome or plasmid DNA) and then become integrated into a different position in the cell genome through transposase enzymatic activity.

DNA transposons have several features, which determine their usefulness as a tool in biotechnology, genome engineering and transgenesis.

Transposons are an effective vector that is used for the introduction of a desired DNA fragment into the cell genome. The transposase, i.e. the enzymatic element of the transposon system, acts in trans on any DNA segment located between the inverted terminal repeats (ITRs) of a transposon. The size of such a segment can be up to several thousand base pairs (piggyBac transposon is ∼70 kbp in size), which exceeds the capacity of the other DNA transfer systems.

Transposons constitute a safer option for viral vectors in therapeutic applications. Unlike the adenoviral vectors, transposons don't evoke responses of the immunological system.

Unlike the gamma retroviruses that have a strong preference for regulator gene sites, most DNA transposons are characterized by a profile of accidental genomic integration, which decreases the risk for undesirable insertions leading to oncogene activation [VandenDriessche T., Ivics Z., Izsvák Z., Chuah M.K.: Emerging potential of transposons for gene therapy and generation of induced pluripotent stem cells. Blood, 2009;114(8):1461-8.].

The very low reprogramming efficiency of 0.0001-0.1 % [Xiaogang Zhang, Alejandro De Los Angeles and Jinqiu Zhang: The Art of Human Induced Pluripotent Stem Cells: The Past, the Present and the Future. The Open Stem Cell Journal, 2010, 2, 2-7.] is a common defect of solutions based on methods without viral integration.

The reprogramming processes described above produce cells that have a low degree of differentiation, which during subsequent steps have to be differentiated into desired cells with different types of factors. The cells can differentiate in an uncontrolled way into different derivative cells, which diminishes the efficiency of the whole process and also results in a migration of these cells to many sites in the organism and in unforeseeable consequences after application to patients.

In the procedure according to the present invention are used unique solutions that considerably improve the obtainment of desired cells. These solutions consist in using two transcription factors - Sox2 and c-Myc. This allows an obtainment of cells that have a different degree of dedifferentiation from that of iPS cells. The described procedure yields iNS cells (induced neural stem cells). Like the reprogramming cassettes, two transcription factors used in a procedure considerably diminish the number of interference sites in the host's genome. Furthermore, the cells are not modified extensively, but only in such a way, which has to correspond with neural stem cells.

Consequently, the risk for neoplastic transformation is low, whereas the employment of the method using integrating vectors allows achievement of reprogramming process high efficiency.

Specialistic literature contains descriptions of reprogramming procedures using several factors simultaneously. Almost complete sets of factors are created and iPS cells are obtained using these sets. The technique that constitutes the subject of the present invention is a unique one, because cells are reprogrammed with two factors (Sox2 and c-Myc) and the dedifferentiation (reprogramming) of cells does not bring about a transformation into iPS cells, but only into a form that has a lower potential for differentiation - the iNS cell (principally, from iPS cells can originate all kinds of cells of the human organism, whereas from iNS cells only glial cells and neurons). An additional, extremely important aspect that ensues from the method according to the present invention is this, that iNS cells are characterized by a considerably lower proneness for neoplastic transformation than iPSCs, which largely enables their therapeutic uses. Like iPS cells, iNS cells must be differentiated to desired stages. Different kinds of factors are used to achieve this effect according to the known methods. The superiority of the new technique consists in this, that the degree of dedifferentiation is not so high as in the procedures with iPS cells. There is no need to use so many substances and very high concentrations in order to differentiate the cells into desired cells. It is only possible to obtain the two described kinds of cells (glial cells and neurons) from NS cells, but due to their lower proneness for neoplastic transformation the range of indications is greater.

So far, employment of one factor in procedures associated with reprogramming was mentioned only once in the literature and this description did not concern reprogramming procedures as in the case of the present invention. It was a description of a cell differentiation procedure. Scientists isolate mesenchymal stem cells from patients' organisms (i.e. simplifying, the scientists leave out the reprogramming and, in their estimation, they collect cells to replace iPSCs obtained by standard reprogramming) and differentiate these cells into neural cells by introducing the factor Nanog. Thus, if one factor is used, the objective is completely different [Angel Alvarez, Monowar Hossain, Elise Dantuma, Stephanie Merchant, Kiminobu Sugaya: Nanog overexpression allows Human Mesenchymal Stem Cells to Differentiate into Neural Cells. Neuroscience & Medicine, 2010, 1, 1-13doi:10.4236/nm.2010.11001.]. However, there is no information about combined use of Sox2 and c-Myc.

The essence of the present invention is a neural stem cell (iNS) that is characterized by an expression of the markers Sox2, GFAP and nestin.

A method for reprogramming somatic cells characterized by the use of two reprogramming factors is also the subject of the present invention.

Advantageously, complete sequences that code for the proteins Sox2 and c-Myc are the reprogramming factors. Reprogramming with nothing but the Sox2 is associated with difficulty in stabilizing Sox2 cells. A standardization of this process is difficult and it fails completely in extreme cases. The conditions of reprogramming using only Sox2 are characterized by low repeatability.

Advantageously, the result of the process of reprogramming somatic cells using two reprogramming factors is the generation of iNS cells that are characterized by the presence of the markers Sox2, nestin (neural stem cell markers) and GFAP (glial fibrillary acidic protein).

Advantageously, the average reprogramming efficiency using two reprogramming factors, i.e. Sox2 and c-Myc, is 0.1%. The process of reprogramming is reproducible.

Advantageously, Sox2-, GFAP- and nestin-positive cells have the ability to differentiate into cells that are characterized by the presence of the markers MAP2+ and beta III+ during culture in NBM and DMEM/F-12 with the differentiation factor bFGF.

The iNS cells obtained with the method according to the present invention are characterized by an easier obtainable and more extensive effect of differentiation into target cells than that of iPS cells.

The iNS cells obtained with the method according to the present invention are also characterized by an absence of an ability to cause teratomas.

An expression of Sox2, nestin and GFAP was observed after a reprogramming using Sox2, but the number of colonies was very low - efficiency of about 0.01%. Unexpectedly, the use of c-Myc factor in the reprogramming procedure allows an achievement of a 10-fold higher reprograming efficiency, even in comparison with the method using one Sox2 factor and the stabilization of iNS cells was easier. Cells obtained with this method are characterized by properties of iNS cells, i.e. the do not cause teratomas.

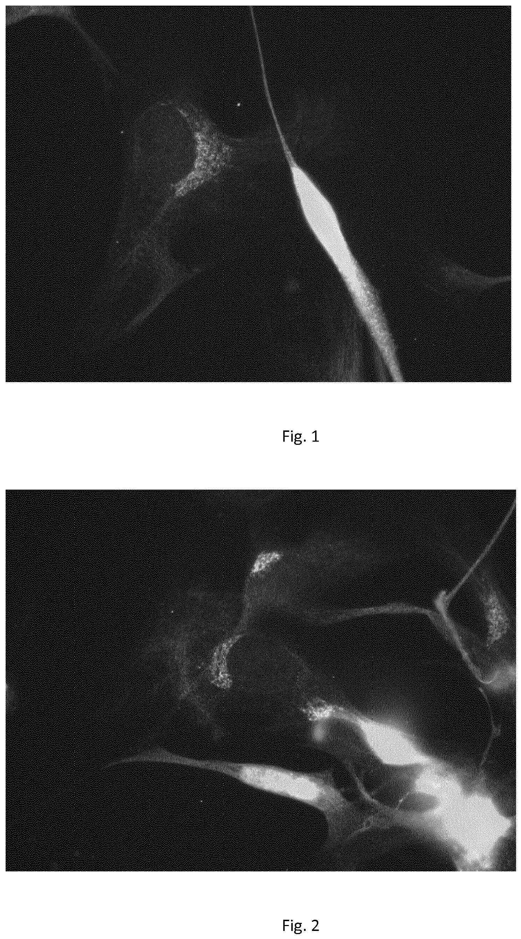

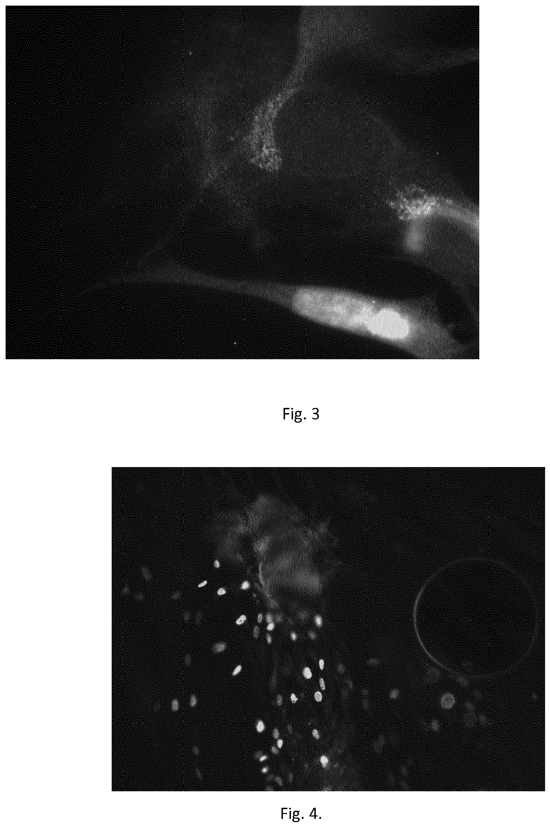

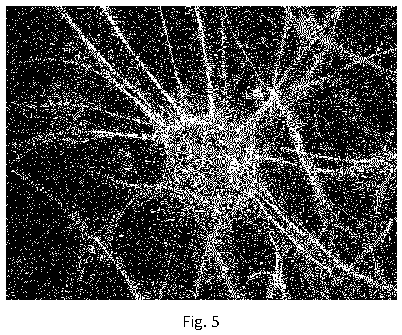

The following examples and specially suitable photographs are illustrative of the present invention: Fig. 1 shows a cell with nuclear expression of Sox2 and GFAP (on the left side); Fig. 2 shows a cell (on the right side) without nuclear expression of Sox2 (nuclear signal in the form of DAPI (4',6-diamidino-2-phenylindole) is visible), whereas a GFAP-negative cell is visible on the right side is; Fig. 3 shows a Sox2-positive cell (bottom, under magnification) with nestin expression; the presence of these two proteins is an evidence of the presence of neural stem cells; Fig. 4 depicts a Sox2 expression in reprogrammed cells with c-MYC and Sox2; and Fig. 5 shows a MAP2 expression in a reprogrammed colony with the two factors Sox2 and c-MYC.

### Examples of Sox2 application

### Example 1.1

### Protocol for generation of iNS cells obtained using the transcription factor Sox2 and lentiviral vector.

Lentiviral vectors distinguish itself by a relatively high capacity (∼8 kbp), a chromosomal integration of transferred genetic material and a long-lasting transgene expression. Unlike the retroviruses, lentiviral vectors transduce also non-dividing cells. Their shortcomings include a risk for insertional mutagenesis.

### 1. Isolation of cellular RNA.

The total cellular RNA was obtained from the AD293 cell line (one 10-cm-diameter dish, ∼5x10⁶ cells). The isolation of RNA was performed using a AxyPrep Midi Kit (AxyGen) according to the enclosed instruction. The concentration of isolated RNA was determined by spectrophotometry (NanoPhotometer, Implen) and the RNA integrity was assessed by by electrophoresis in an agarose gel stained with ethidium bromide (∼500 ng of RNA; the gel was checked for the presence of bands corresponding with 18S and 5S rRNA). The isolated RNA was stored at -80°C.

### 2. Reverse transcription reaction and cDNA synthesis.

The isolated RNA was used as a template for reverse transcription. The reaction was performed with M-MuLV reverse transcriptase (NEB), according to the enclosed protocol, using an oligo(dT)18 primer hybridizing with mRNA polyadenylation signal that was used as a primer for complementary DNA (cDNA) synthesis. A mixture containing 5µg of total RNA, 500 ng of oligo(dT) 18, and 1 µl of 10mM dNTPs was incubated at 65°C for 5 min to eliminate secondary structures and after a cooling, it was supplemented with a reaction buffer, DDT, a protein RNase inhibitor (NEB) and 200 units of reverse transcriptase. The reaction mixture was incubated at 50°C for 60 min and then the temperature was raised to 75°C (15 min) to inactivate the enzyme. The synthesized cDNA was stored at -80°C.

### 3. Amplification of Sox2 coding sequence.

The complementary DNA (cDNA) was used as a template for transcription factor Sox2 coding sequence amplification. The PCR reaction was performed with a Pfu Ultra II repair activity DNA polymerase (Stratagene) and the following primer pair:

| | |
|---|---|
| Sox2 NheI Fwd | 5'-GACT GCTAGC CGCCACC ATG TAC AAC ATG ATG GAG ACG GAG CT-3'; |
| Sox2 BamHI Rev | 5'-GACT GGATCC TCA CAT GTG TGA GAG GGG CAG TG-3'. |

The following reaction mixture,

| | |
|---|---|
| reaction buffer - 10× | 5 µl |
| dNTPs (each dNTP 25 mM) | 0.5 µl |
| template (cDNA from the previous step) | 1 µl |
| Sox2 primer Nhel Fwd (10 µM) | 1 µl |
| Sox2 primer BamHI Rev (10 µM) | 1 µl |
| Pfu II polymerase | 1 µl |
| H₂O | 40.5 µl |

was used for PCR amplification according to the following programme:

| | | | | | |
|---|---|---|---|---|---|
| 1. | Denaturation | 95°C | 1 min | | |
| 2. | Denaturation | 95°C | 20 s | 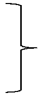 | |
| | Annealing | 55°C | 20 s | | 40 cycles |
| | Elongation | 72°C | 30 s | | |
| 3. | Final elongation | 72°C | 3 min. | | |

A PCR reaction product separation in agarose gel was performed and the band corresponding with the size of complete coding sequence (∼960 bp) was cut out from the gel. DNA was isolated using the AxyPrep Gel Extraction Kit (AxyGen) and its concentration was determined by spectrophotometry (NanoPhotometer, Implen).

### 4. Sox2 factor cloning into pIRESneo3 vector

2 µg of pIRESneo3 plasmid (Clontech) and 1 µg of the PCR product containing the Sox2 sequence were digested with NheI/BamHI restriction enzymes. Then, the DNA vector was dephosphorylated with antarctic phosphatase (NEB) according to the enclosed instruction. The DNA was separated in an agarose gel and the bands of which size corresponded to an empty vector and to the Sox2 insert were isolated and cleared of the gel. The ligation reaction of vector to Sox2 insert was performed with T4 DNA ligase (NEB) at 12°C for 16 h. 50 ng of vector, 200 ng of insert and 0.5 µl of enzyme were used for the reaction. The ligation product was introduced into chemically competent *E. coli* cells. An expansion of a number of isolated bacterial colonies in a LB medium with ampicillin was carried out and then a DNA isolation from these bacterial colonies, digestion with NheI/BamHI restrictive enzymes and sequencing were performed to confirm the presence of complete Sox 2 ORF and absence of mutations.

The following primers were used for sequencing:

| | |
|---|---|
| CMV seq primer | 5'- CGC AAA TGG GCG GTA GGC GTG-3'; |
| hSox2 seq primer #2 | 5'- TTA CGC GCA CAT GAA CGG CTG G-3'. |

### 5. Subcloning of the CMV-hSox2-IVS-IRES fragment into the pLOC lentiviral vector

2 µg of pIRESneo3-hSox2 plasmid (containing an insert) were digested with Ndel/BfrBI restriction enzymes. 2 µg of pLOC-RFP vector coding for red fluorescent protein variant under CMV promoter control were digested with Nhel restriction enzyme. The sticky ends of the undergoing digestion vector were filled with Klenow fragment in the presence of 1mM dNTPs and a reaction buffer from the enzyme supplier. The reaction was terminated after 30 min by a supplementation with EDTA (up to 100 mM) and thermal inactivation (65°C, 10 min). Then, the DNA was purified using AxyPrep PCR Cleanup Kit (AxyGen) and digested with Ndel restriction enzyme. A separation of the DNAs of the undergoing digestion plasmid containing CMV-hSox-IVS-IRES insert and of the undergoing digestion lentiviral vector filled up with Klenow polymerase was performed in agarose gel. Bands corresponding with the empty vector (∼8.8 kbp) and insert (∼1.7 kbp) were isolated and cleared of the gel using an AxyPrep Gel Extraction Kit (AxyGen). In order to obtain a complete lentiviral vector, a ligation of the isolated DNA was performed. 50 ng of vector, 200 ng of insert and 0.5 µl of ligase were used for the reaction. The reaction was performed at 12°C for 16 h and the ligation product was introduced into chemically competent *E. coli* cells. An expansion of a number of isolated bacterial colonies in a LB medium with ampicillin, a DNA isolation from these colonies and test digestion with NdeI/EcoRI restrictive enzymes to confirm the presence of a correct insert (∼7.3 kbp and 3 kbp fragments) were carried out.

### 6. Transfection of packing HEK293T cells with pLOC-hSox2 vector

The dishes intended for maintaining packaging cells were coated with poly-L-lysine (5 ml of 0.0005% solution for ∼60 min). The HEK293T cells (6×10⁶ cells in each dish) were placed in a DMEM culture medium and an uniform cell distribution was ensured. These cells were maintained at 37°C in an atmosphere of 5% CO₂. The next day, the cells were washed with PBS and 10 ml of fresh culture medium were added. A transfection of HEK293T packaging cells was performed using PEI (polyethylenimine; Polysciences). A transfection in one 10-cm-diameter dish was carried out according to the following scheme: 60 µl of PEI (1 µg/µl) were added to 640 µl of 150 mM NaCl contained in an 1.5-ml tube. 5 µg of pLOC-hSox2 vector were mixed with 10 µg of packaging plasmids (containing equal amounts of pTLA1-Pak, pTLA1-Enz, pTLA1-Env, pTLA1-TetOFF and pTLA1-Tat/Rev) in a tube and then the mixture was filled up to the final volume of 700µl with 150 mM NaCl. Afterwards, the PEI solution in NaCl was added to the tube containing DNA, the contents were mixed, centrifuged and left for incubation at room temperature for 30 minutes. The solution containing PEI/DNA complexes was then transferred drop by drop to a medium with cultured HEK 293T cells. The dish was placed in an incubator for 8-12 h. The following day, the culture medium was removed and replaced with a DMEM solution (with glucose and antibiotics) without serum. Then, the cells were maintained under such conditions for 40-48 h. During this period, the transfection efficiency was also assessed on the grounds of fluorescent reporter expression.

### 7. Isolation of lentiviruses.

In order to isolate the virus, the culture medium was collected and centrifuged (3000 rpm, 10 min, 4°C) to remove dead cell remnants. Then, the medium containing viruses was filtered (a 0.45-µm filter, washed with PBS) and intended for ultracentrifugation. The cellular medium was centrifuged for 3 h (50000 G, 4°C) or 18 h (20000 G, 4°C). After centrifugation, the supernatant was removed and the viral pellet was suspended in sterile PBS (1:300 of original medium volume). The lentiviruses obtained by this method were separated into 1.5 ml tubes (20-50 µl into each tube) and stored at -80°C. The transduction biological efficiency was checked in HEK293 cells and lentiviruses (and their concentrations), which produced more than 40% GFP-positive infected cells, were intended for further use.

### 8. Generation of iNS cells from fibroblasts/keratinocytes with a prepared lentivirus

Fibroblastic cells and keratinocytes taken from patients were infected with obtained lentiviruses. Each cell line was transduced with a hSox2 coding virus. The harvested cells were maintained in MEM supplemented with 10% fetal bovine serum. After achieving a 40-80% confluence, the cells were infected. After a transduction wtih the virus (48 h), the culture medium was changed to a medium supporting reprogramming to iNS cells and a selction of the cells with a medium supplemented with blasticidin S (5 µg/ml). Following a one-week's selection, a morphological assessment of the cells and immunohistochemical analysis of specific marker expression was carried out. During the three weeks following selection, colonies of cells reprogrammed to iNS cells were transferred on a layer of fibroblasts treated with mitomycin (feeder layer) in an ESC (embryonic stem cell) medium. After about one month, a differentiation of iNS cells into neuronal and glial cells was performed.

### Example 1.2

### The protocol for generation of iNS cells obtained using the transcription factor Sox2 and an adenoviral vector

Adenoviral vectors are characterized by a high transduction efficiency, broad spectrum of tissue specificity, heterologous gene expression of up to ∼7 kbp, absence of chromosomal integration, transient transgene expression and a possibility of achieving a high titre of isolated virions. However, adenovirus infections induce a strong response of the immunological system.

The steps 1-4 are identical with the steps in Example 1:
1. Isolation of cellular RNA.
2. Reverse transcription reaction and obtainment of cDNA.
3. Amplification of the Sox 2 coding sequence.
4. Cloning of the Sox2 factor into the pIRESneo3 vector.
5. Mutagenesis of the pShuttle-CMV-LacZ vector.

Site-specific mutagenesis of the shuttle vector, intended for cloning of insert containing hSox2 sequence, was performed to facilitate further procedures with DNA. Modification of the NdeI restriction site (cutting site 7745) in the pShuttle-CMV-LacZ vector by exchanging two nucleotides was performed using the following primers:

| | |
|---|---|
| pShuttle mut Fwd CAC -3'; | 5'- AAT TAA CAT GCA TGG ATC CAT GCG CGG TGT GAA ATA CCG |
| pShuttle mut Rev | 5'-GTG CGG TAT TTC ACA CCG CGC ATG GAT CCA TGC ATG TTA ATT -3'. |

Primer elongation reaction was performed with a Pfull Ultra HS repair activity DNA polymerase (Stratagene) according to the following mutagenesis programme:

| | | | | |
|---|---|---|---|---|
| 1. | Denaturation | 95°C 1 min | | |
| 2. | Denaturation | 95°C 50 s | 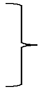 | |
| | Annealing | 60°C 50 s | | 18 cycles |
| | Elongation | 68°C 11 min | | |
| | | | | |
| 3. | Final elongation | 68°C 7 min | | |

The reaction product was digested with Dpnl restriction enzyme that acts on methylated bacterial plasmid DNA, which makes it possible to collect the digestion product without removing the non-methylated mutagenesis product. After purification (AxyPrep PCR Cleanup, AxyGen), the mutant plasmid was transformed and propagated in *E. coli* cells. The mutagenesis reaction correctness was confirmed by an analysis using Ndel restriction enzyme and electrophoretic analysis of digested DNA.

### 6. Subcloning of the CMV-hSox2-IRES-tGFP(2A)Blast-WPRE fragment into the pShuttle vector

2 µg of pShuttle-CMV-LacZ(mut) plasmid were digested with Ndel/Blpl restriction enzymes and then dephosphorylated with antarctic phosphatase (NEB). 2 µg of lentiviral plasmid (pLOC-hSox2) containing the CMV-hSox2-tGFP(2A)Blast-WPRE fragment were digested with NdeI/Bsu36I enzymes. Afterwards, a DNA separation in an agarose gel was performed and bands corresponding with the vector (∼7.3 kbp) and insert (∼4.3 kbp) were isolated and cleared of the gel (AxyPrep Gel extraction, AxyGen). A ligation reaction of insert containing Sox2 sequence to shuttle vector was performed using T4 DNA ligase (NEB) at 12°C for 16 h. 50 ng of vector, 200 ng of insert and 0.5 µl of enzyme were used for the reaction. The ligation product was introduced into chemically competent *E. coli* cells. An expansion of a number of isolated bacterial colonies in an LB medium with ampicillin was carried out and then a DNA isolation and digestion with XhoI/NotI restriction enzymes to confirm the presence of a complete insert (expected: 2 bands - 10 kbp and 1.6 kbp) were performed.

### 7. Homologous recombination in vivo between the pShuttle vector and the adenoviral vector pAdEasy-1

In order to obtain a complete adenoviral vector, a homologous recombination between pShuttle-hSox2 shuttle plasmid and E1-defective vector pAdEasy-1 containing the other part of the adenoviral genome was performed. The electrocompetent *E. coli* strain BJ5183-AD-1 (pretransformed with a Stratagene plasmid pAdEasy-1) that contains the native recA gene was used for the recombination. The pShuttle-hSox2 plasmid was linearized with Pmel restriction enzyme and then ∼100 ng of it were added to the bacterial suspension. The electroporation parameters were: 200 Ω, 2.5 kV, 25 µF. After an ∼1h incubation in a SOC medium at 37°C, the cells were transferred into a selective agar medium with kanamycin. The following day, 10 bacterial colonies were selected and after an ∼8h incubation and expansion in a LB medium with kanamycin DNA was isolated (AxyPrep Plasmid Miniprep, Axygen). Recombinant adenoviral vectors were identified by restriction analysis using Pacl enzyme (expected: 2 products - ∼32 kbp and 3 kbp).

The DNA obtained from positively identified colonies was then used for a transformation of recA mutant and end1-negative chemically competent *E. coli* cells (Turbo, NEB).

Afterwards, a plasmid containing recombinant complete adenoviral genome was isolated and purified (using AxyPrep Plasmid Maxiprep, Axygen) from these *E. coli* cells.

### 8. Transfection of AD-293 packaging cells and generation of initial adenoviral plaques

Dishes intended for packaging cell cultures were coated with poly-L-lysine (5 ml of 0.0005% solution for ∼60 min). AD-293 cells were transferred into a DMEM culture medium at a volume of 6x10⁶ cells per addition to a dish and their uniform distribution was ensured. These cells were maintained at 37°C in an atmosphere of 5% CO₂. The next day, the cells were washed with PBS and 10 ml of fresh culture medium were added. The transfection of AD-293 packaging cells was performed using PEI (Polyethylenimine, Polysciences). DNA of a complete adenoviral vector linearized with Pacl restriction enzyme containing the hSox2 sequence was used for the transfection. Transfection in one 10-cm-diameter dish was carried out according to the following scheme: 20µl of PEI (1µ g/µl) were added to 680 µl of 150 mM NaCl in a 1.5-ml tube. 5µg of a linearized vector were placed in a tube and the contents were filled up to the final volume of 700 µl with 150 mM NaCl. Then, a PEI solution in NaCl was added to the tube containing DNA, the contents were mixed, centrifuged and left for a 30 minute incubation at room temperature. Afterwards, the solution containing PEI/DNA complexes was transferred drop by drop into the AD-293 cell culture medium. The dish was placed in an incubator for 12 h. The following day, the culture medium was removed and the cells were gently coated with 3.5ml of 1% low-melting agarose (heated to 42°C) in a culture medium. When the agarose has solidified, 7 ml of DMEM (with glucose and antibiotics) were added to each dish. The cells were maintained in an incubator at 37°C in an atmosphere of 5% CO₂. After 5 days, the liquid medium was removed and the next layer consisting of 3.5 ml of 1% agarose was placed in DMEM. Adenoviral plaques should be observable 7-10 days after transfection.

### 9. Initial adenoviral plaque expansion in AD-293 packaging cells

Ten days after transfection, the adenoviral plaques were aspirated using pipettes with sterile 1-ml tips. The plaques were suspended in 0.5 ml of PBS and four cycles of freezing-defreezing (-80°C - 37°C) were performed to release the adenovirus. In order to amplify the adenovirus, 500 µl of a suspension containing primary viruses were added to a T150 culture flask medium containing AD-293 packaging cells (at ∼70% confluence). The infected cells were maintained in an incubator at 37°C in an atmosphere of 5% CO₂ for 5-7 days till the appearance of observable cell lysis. The collected culture medium (containing cells) underwent four cycles of freezing-defreezing (-80°C - 37°C) to release the adenovirus and then it was centrifuged (at 3000 rpm, 4°C, 5 min) to separate cell remnants. The harvested supernatant (crude extract) was used for isolation of adenoviruses.

### 10. Isolation and purification of adenoviruses.

Adenoviruses were isolated using caesium chloride gradient ultracentrifugation. 2.5 ml of a CsCI solution (δ=1.4 g/ml) in TBS were placed in a 50-ml ultracentrifugation tube. The upper layer was formed by a CsCI solution with a density δ=1.25 g/ml. 5 ml of crude adenoviral extract were added to the CsCI gradient and then it was coated with 2 mm of mineral oil to avoid aerosolization and apparatus contamination with the virus. The samples prepared by this method were centrifuged (35000 rpm, 1h, 15°C). After centrifugation, the white band at the CsCI gradient phase boundary was collected using a disposable syringe with a 20G needle (the lower and upper band was formed by proteins of an empty adenoviral capsid). The isolated adenovirus was dialyzed in a 4% glucose solution in PBS and the obtained product was then portioned 20-50 µl by 20-50 µl and stored at -80°C. Biological transduction efficiency was determined in HEK293T cells and viruses (and their concentrations), which produced at least 50% GFP-positive infected cells were intended for further use.

### 11. Generation of iNS cells from fibroblasts/keratinocytes using a prepared adenovirus

Like the lentiviral vector that is the source of hSox2 factor expression, fibroblastic cells and keratinocytes collected from patients were infected with the obtained adenoviruses. Each of the cell lines was transduced with hSox2 coding virus. The collected cells were maintained in a MEM supplemented with 10% fetal bovine serum. After achieving a 40-80% confluence, these cells were infected. After viral transduction (48h), the culture medium was changed to a medium supporting reprogramming to iNS cells. A selection of cells was performed using a medium supplemented with blasticidin (5µg/ml). After a one-week's selection, a morphological assessment of the cells and immunohistochemical analysis of specific marker expression was carried out. During subsequent three weeks, colonies of cells reprogrammed to iNS cells were transferred on a layer of fibroblasts treated with mitomycin (feeder layer) in a ESC (embryonic stem cells) medium. One month later a differentiation of iNS cells into glial and neuronal cells was performed.

### Example 1.3

### Protocol for generation of iNS cells using the transcription factor Sox2 and a retroviral vector

Like lentiviruses, retroviral vectors are distinguishable by a high capacity (∼8 kbp), chromosomal integration of the transferred genetic material and long-lasting transgene expression. However, these vectors do not transduce non-dividing cells. Retroviral vectors are known to be associated with a risk for insertional mutagenesis and show preferential integration into transcriptionally active regions. Unlike lentiviral vectors pseudotypified with VSV-G protein (pantropic lentiviruses), ampho- and dualtropic packaging plasmids that are characterized by a different cell transduction mechanism (through Ram-1 and GALV receptors) were used in the procedures according to the present protocol.

The steps 1-4 are identical with the steps in example 1:
1. Isolation of cellular RNA.
2. Reverse transcription reaction and obtainment of cDNA.
3. Amplification of the Sox2 coding sequence.
4. Cloning of the Sox2 factor into the pIRESneo3 vector.
5. Subcloning of the hSox2-IRES-tGFP(2A)Blast-WPRE fragment into the pFB retroviral vector

2 µg of pFB plasmid was digested EcoRI/Notl restriction enzymes, incubated with mung bean nuclease and dephosphorylated using antarctic phosphatase (NEB). 2 µg of lentiviral plasmid (pLOC-hSox2) containing the hSox2-tGFP(2A)Blast-WPRE fragment were digested with ClaI/Bsu36I enzymes, incubated with mung bean nuclease to obtain blunt ends and phosphorylated with T4 polynucleotide kinase. A DNA separation in agarose gel was performed and bands corresponding with the vector (∼5.3 kbp) and insert (∼3.7 kbp) were isolated and cleared of the gel (AxyPrep Gel extraction, AxyGen). A ligation reaction of insert containing Sox2 sequence to shuttle vector was performed using T4 DNA ligase (NEB) at 12°C for 16 h. 50 ng of vector, 200 ng of insert and 0.5 µl of enzyme were used in this reaction. The ligation product was introduced into chemically competent *E. coli* cells. An expansion of a number of isolated bacterial colonies in an LB medium with ampicillin was carried out and then a DNA isolation and digestion with Bsu36I/BamHI restriction enzymes to confirm the correctness of insert orientation (expected: 2 bands - 7.2 kbp and 1.7 kbp) was performed.

### 6. Transfection of HEK293T packaging cells with the pFB-hSox2 vector

Dishes intended for maintaining packaging cells were coated with poly-L-lysine (5 ml of 0.0005% solution for ∼60 min). HEK293T cells were transferred into a DMEM culture medium (6x10⁶ cells on each dish) and their uniform distribution was ensured. These cells were maintained at 37°C in an atmosphere of 5% CO₂. The next day, the cells were washed with PBS and 10 ml of fresh culture medium were added. The transfection of HEK293T packaging cells was performed using PEI (Polyethylenimine, Polysciences). Transfection in one 10-cm-diameter dish was carried out according to the following scheme: 40µl of PEI (1µg/µl) were added to 660 µl of 150 mM NaCl in a 1.5-ml tube. 3 µg of pFB-hSox2 vector were mixed with 3µg of pVPack-GP and 3µg of envelope protein coding plasmids (pVPack-Ampho or pVPack-10A1) in a tube and the contents were filled up to the final volume of 700µl with 150 mM NaCl. Then, a PEI solution in NaCl was added to the tube containing DNA, the contents were mixed, centrifuged and left for a 30 minute incubation at room temperature. Afterwards, the solution containing PEI/DNA complexes was transferred drop by drop into the HEK 293T cell culture medium. The dish was placed in an incubator for 8-12 h. The following day, the culture medium was removed and replaced with a DMEM solution (with glucose and antibiotics) without serum. The cells were maintained under this conditions for 40-48 h. During this period, transfection efficiency was assessed on the grounds of fluorescent reporter expression.

### 7. Isolation of retroviruses.

In order to isolate the virus, the culture medium was collected and centrifuged (3000 rpm, 10 min, 4°C) to remove dead cell remnants. Then, the medium containing retroviruses was filtered (a 0.45-µm filter, washed with PBS) and intended for ultracentrifugation. The cellular medium was centrifuged for 3 h (50000 G, 4°C) or 18 h (20000 G, 4°C). After centrifugation, the supernatant was removed and the viral pellet was suspended in sterile PBS (1:300 of original medium volume). The obtained retroviruses were separated into 1.5 ml tubes (20-50 µl into each tube) and stored at-80°C. The transduction biological efficiency was checked in HEK293 cells and viruses (and their concentrations), which produced more than 40% GFP-positive infected cells were intended for further use.

### 8. Generation of iNS cells from fibroblasts/keratinocytes using a prepared retrovirus

Fibroblastic cells and keratinocytes collected from patients were infected with obtained retroviruses. A transduction of each of the cell lines with hSox2 coding virus was performed. The collected cells were maintained in a MEM medium supplemented with 10% fetal bovine serum. After achieving a 40-80% confluence, these cells were infected. After viral transduction (48h), the culture medium was changed to a medium supporting reprogramming to iNS cells and a selection of cells with a medium supplemented with blasticidin (5 µg/ml) was performed. After a one-week's selection, a morphological assessment of the cells and immunohistochemical analysis of specific marker expression were carried out. During subsequent three weeks, colonies of cells reprogrammed to iNS cells were transferred on a layer of fibroblasts treated with mitomycin (feeder layer) in a ESC (embryonic stem cells) medium. About one month later a differentiation of iNS cells into glial and neuronal cells was performed.

### Example 1.4

### Protocol for generation of iNS cells using the transcription factor and piggyback transposon vector

The piggyBac transposon vector is characterized by a high capacity (up to 100 kbp), chromosomal integration of transferred genetic material and long-lasting transgene expression. The essential feature of it is the possibility to remove the vector from the genome through transfection of induced cells using a transposase coding plasmid. This leads to a restoration of original cell genotype, which is of importance for cell lines obtained with this method and intended for therapeutic use.

The steps 1-4 are identical with the steps in example 1:
1. Isolation of cellular RNA.
2. Reverse transcritpion reaction and obtainment of cDNA.
3. Amplification of the Sox2 coding sequence.
4. Cloning of the Sox2 factor into the pIRESneo3 vector.
5. Subcloning of the CMV-hSox2-IRES-tGFP(2A)Blast-WPRE fragment into the site between the inverted terminal repeats of a transposon.

2 µg of pPB51x-EF1α-Puro plasmid (SBI) were digested with Ascl/Fsel restriction enzymes (located outside the HS4 insulator sequence), incubated with mung bean nuclease and dephosphorylated with antarctic phosphatase (NEB). 2 µg of lentiviral plasmid (pLOC-hSox2) containing CMV-hSox2-tGFP(2A)Blast-WPRE fragment were digested with SanDI/Bsu36I enzymes, incubated with mung bean nuclease to obtain blunt ends and phosphorylated with T4 polynucleotide kinase. The DNA was separated in agarose gel and bands corresponding with the vector (∼5.5 kbp) and the insert (∼4.5) were isolated and cleared of the gel (AxyPrep Gel extraction, AxyGen). The ligation reaction of piggyBac vector and insert containing Sox2 sequence was performed with T4 DNA ligase (NEB) under the control of CMV promoter at 12°C for 16 h. 50 ng of vector, 200 ng of insert and 0.5 µl of enzyme were used for the reaction. The ligation product was introduced into chemically competent *E. coli* cells. An expansion of a number of isolated bacterial colonies was performed in a LB medium with ampicillin and then a DNA isolation and restriction analysis were carried out.

### 6. Transfection of primary fibroblastic cells/keratinocytes with a prepared piggyBac vector and transposase coding plasmid

Cultured fibroblastic cells and keratinocytes from patients underwent transfection with a prepared transposon and plasmid coding for the enzymatic component - transposase (Super PiggyBac transposase plasmid (SBI) coding for optimized enzyme variant). To avoid an effect of reaction inhibition caused by defective enzyme variants (overproduction inhibition), the DNA was introduced at 1:5 ratio (transposase/transposon) that is optimal for this system. The transfection was performed with the FuGENE® HD (Roche) reagent. 9 µg of DNA (1.5 µg of piggyBac transposase coding plasmid and 7.5 µg of transposon) were transferred into 700 µl of DMEM (without antibiotics and serum) culture medium.

Then, 18 µl of the FuGENE® reagent were added and the medium was filled up to the volume of 800 µl, mixed and left at room temperature for 15 min. Fibroblasts and keratinocytes were maintained in 10-cm-diameter dishes. The transfection was performed with cells located above the layer with 50% confluence. The FuGENE®/DNA mixture was transferred drop by drop into a cell culture medium, which was placed in an incubator for 48 h.

### 7. Selection of cells with a chromosomally integrated transpozon and generation of iNS cells

Two days after the transfection, the cellular medium was removed and replaced with a medium supporting reprogramming to iNS cells. A selection of cells with blasticidin (5 µg/ml) was started simultaneously. After a one-week's selection, a morphological assessment of cells and immunohistochemical analysis of specific marker expression were carried out

### 8. Transposon remobilization

The integrated transposon was removed through transfection of reprogrammed cells with a plasmid coding only for transposase. A transfection was performed with 10 µg of Super PiggyBac transposase plasmid (SBI) in a 10-cm-diameter dish and the contents were transferred into 700 µl of DMEM culture medium (without added antibiotics and serum). Then, 20 µl of FuGENE® transfection reagent were added and the medium was filled up to the volume of 800 µl, mixed and left at room temperature for 15 min. Afterwards, the FuGENE®/DNA mixture was transferred drop by drop into the medium with cultured cells and without blasticidin, which was placed in an incubator for 48 h. The transposon remobilization (loss) was assessed on the grounds of reprogrammed cell fluorescence decay (caused by a tGFP(2A)Blast cassette). A morphological assessment of the cells obtained by this method and immunohistochemical analysis of specific marker expression were carried out. During subsequent three weeks, colonies of cells reprogrammed to iNS cells were transferred on a layer of fibroblasts treated with mitomycin (feeder layer) in a ESC medium (embryonic stem cells). About one month later, a differentiation of iNS cells into glial and neuronal cells was performed.

Examples of reprogramming by using the two reprogramming factors Sox2 i c-Myc

### Example 2.1

### Protocol for generation of iNS cells using the transcription factors Sox2 and c-Myc and a lentiviral vector

### 1. Isolation of cellular RNA.

The total cellular RNA was obtained from the AD293 cell line (one 10-cm-diameter dish, ∼5x10⁶ cells). Izolacj 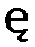 RNA przeprowadzono przy pomocy zestawu AxyPrep Midi (AxyGen), wed 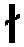 ug za 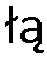 czonej instrukcji. The isolation of RNA was performed using a AxyPrep Midi Kit (AxyGen) according to the enclosed instruction. The concentration of isolated RNA was determined by spectrophotometry (NanoPhotometer, Implen) and the RNA integrity was assessed by electrophoresis in an agarose gel stained with ethidium bromide (∼500 ng of RNA; the gel was checked for the presence of bands corresponding with 18S and 5S rRNA). The isolated RNA was stored at -80°C.

### 2. Reverse transcription reaction and obtainment of cDNA.

The isolated RNA was used as a template for reverse transcription. The reaction was performed with M-MuLV reverse transcriptase (NEB), according to the enclosed protocol, using an oligo(dT)18 primer hybridizing with mRNA polyadenylation signal that was used as a primer for complementary DNA (cDNA) synthesis. The mixture containing 5µg of the total RNA, 500 ng of oligo(dT) 18, and 1 µl of 10mM dNTPs was incubated at 65°C for 5 min to eliminate secondary structures and after a cooling, it was supplemented with a reaction buffer, DDT, a protein RNase inhibitor (NEB) and 200 units of reverse transcriptase. The reaction mixture was incubated at 50°C for 60 min and then the temperature was raised to 75°C (15 min) to inactivate the enzyme. The synthesized cDNA was stored at -80°C.

### 3. Amplification of the Sox2 coding sequence and c-Myc coding sequence

The complementary DNA (cDNA) was used as a template for amplification of the sequence coding for the transcription factors Sox2 and c-Myc. The PCR reaction was performer with a Pfu Ultra II repair activity DNA polymerase (Stratagene) and the following primer pair:

| | |
|---|---|
| Sox2 NheI Fwd | 5'-GACT GCTAGC CGCCACC ATG TAC AAC ATG ATG GAG ACG GAG CT-3' |
| Sox2 BamHI Rev | 5'-GACT GGATCC TCA CAT GTG TGA GAG GGG CAG TG-3' |
| c-Myc NheI Fwd. | 5'-GACT GCTAGC CGC CAC CAT GGA TTT TTT TCG GGT AGT GGA AAA CCA GC-3' |
| c-Myc BamHI Rev. | 5'-GACT GGATCC TTA CGC ACA AGA GTT CCG TAG CTG TTC AAG-3'. |

The following reaction mixture,

| | |
|---|---|
| reaction buffer - 10× | 5µl |
| dNTPs (25mM of each dNTP) | 0.5µl |
| template (cDNA from the previous step) | 1µl |
| Fwd primer (10 µM) | 1µl |
| Rev primer (10 µM) | 1µl |
| Pfu II polymerase | 1µl |
| H₂O | 40.5µl |

was used for PCR amplification according to the following programmes:

**(Sox2)**

| | | | | | |
|---|---|---|---|---|---|
| 1. | Denaturation | 95°C | 1 min | | |
| 2. | Denaturation | 95°C | 20 s | } | |
| | Annealing | 55°C | 20 s | | 40 cycles |
| | Elongation | 72°C | 30 s | | |
| 3. | Final elongation | 72°C | 3 min | | |

**(c-Myc)**

| | | | | | |
|---|---|---|---|---|---|
| 1. | Denaturation | 95°C | 1 min | | |
| 2. | Denaturation | 95°C | 20 s | } | |
| | Annealing | 55°C | 20 s | | 40 cycles |
| | Elongation | 72°C | 45 s | | |
| 3. | Final elongation | 72°C | 3 min | | |

A separation of the PCR reaction product in an agarose gel was performer and the band corresponding with the size of complete coding sequence (∼960 bp for Sox2 and ∼1370 bp for c-Myc) was cut out from the gel. The DNA was isolated using the AxyPrep Gel Extraction Kit (AxyGen) and its concentration was determined by spectrophotometry (NanoPhotometer, Implen).

### 4. Cloning of transcription factor sequences into the pIRESneo3 vector

2 µg of pIRESneo3 plasmid (Clontech) and 1 µg of the PCR product containing the Sox2 sequence were digested with NheI/BamHI restriction enzymes. Then, the DNA vector was dephosphorylated with antarctic phosphatase (NEB) according to the enclosed instruction. The DNA was separated in an agarose gel and the bands of which size corresponded to an empty vector and to the inserts Sox2 and c-Myc were isolated and cleared of the gel. The ligation reaction of vector to insert was performed with T4 DNA ligase (NEB) at 12°C for 16 h. 50 ng of vector, 200 ng of insert and 0.5 µl of enzyme were used for the reaction. The ligation product was introduced into chemically competent *E. coli* cells. An expansion of a number of isolated bacterial colonies in a LB medium with ampicillin was carried out and then a DNA isolation from these bacterial colonies, digestion with NheI/BamHI restrictive enzymes and sequencing were performed to confirm the presence of complete Sox 2 and c-Myc ORF and absence of mutations.

The following primers were used for plasmid sequencing:

| | |
|---|---|
| CMV seq primer | 5'- CGC AAA TGG GCG GTA GGC GTG-3'; |
| pIRESneo3 rev | 5'- GAG TAC TCA CCC CAA CAG -3. |

### 5. Subcloning of the CMV-hSox2-IVS-IRES fragment and CMV-c-Myc-IVS-IRES fragment into the pLOC lentiviral vector

2 µg of pIRESneo3-hSox2 plasmid and 2 µg of pIRESneo3-c-Myc plasmid (both containing an insert) were digested with Ndel/BfrBI restriction enzymes. 2 µg of pLOC-RFP vector coding for red fluorescent protein variant under CMV promoter control were digested with Nhel restriction enzyme. The sticky ends of the undergoing digestion vector were filled with Klenow fragment in the presence of 1mM dNTPs and a reaction buffer from the enzyme supplier. The reaction was terminated after 30 min by a supplementation with EDTA (up to 100 mM) and thermal inactivation (65°C, 10 min). Then, the DNA was purified using AxyPrep PCR Cleanup Kit (AxyGen) and digested with Ndel restriction enzyme. The DNAs of the undergoing digestion plasmids that contain transcription factors and of the undergoing digestion lentiviral vector filled up with Klenow polymerase were separated in agarose gel. Bands corresponding with the empty vector (∼8.8 kbp) and insert (∼1.7 kbp) were isolated and cleared of the gel using an AxyPrep Gel Extraction Kit (AxyGen). In order to obtain a complete lentiviral vector, a ligation of the isolated DNA was performed. 50 ng of vector, 200 ng of insert and 0.5 µl of ligase were used in the reaction. The reaction was performed at 12°C for 16 h and the ligation product was introduced into chemically competent *E. coli* cells. An expansion of a number of isolated bacterial colonies in a LB medium with ampicillin, a DNA isolation from these colonies and test digestion with NdeI/EcoRI restrictive enzymes to confirm the presence of a correct insert (∼7.3 kbp and 3 kbp fragments) were carried out.

### 6. Transfection of HEK293T packaging cells with the pLOC-hSox2 vector and c-Myc vector

The dishes intended for maintaining packaging cells were coated with poly-L-lysine (5 ml of 0.0005% solution for ∼60 min). The HEK293T cells were placed in a DMEM culture medium at a volume of 6x10⁶ cells per addition to a dish and an uniform cell distribution was ensured. These cells were maintained at 37°C in an atmosphere of 5% CO₂. The next day, the cells were washed with PBS and 10 ml of fresh culture medium were added. A transfection of HEK293T packaging cells was performed using PEI (polyethylenimine; Polysciences). A transfection in one 10-cm-diameter dish was carried out according to the following scheme: 60 µl of PEI (1 µg/µl) were added to 640 µl of 150 mM NaCl contained in an 1.5-ml tube. 5 µg of pLOC-hSox2 vector were mixed with 10 µg of packaging plasmids (containing equal amounts of pTLA1-Pak, pTLA1-Enz, pTLA1-Env, pTLA1-TetOFF and pTLA1-Tat/Rev) in a tube and then the mixture was filled up to the final volume of 700µl with 150 mM NaCl. Afterwards, the PEI solution in NaCl was added to the tube containing DNA, the contents were mixed, centrifuged and left for incubation at room temperature for 30 minutes. The solution containing PEI/DNA complexes was then transferred drop by drop to a medium with cultured HEK 293T cells. The dish was placed in an incubator for 8-12 h. The following day, the culture medium was removed and replaced with a DMEM solution (with glucose and antibiotics) without serum. Then, the cells were maintained under such conditions for 40-48 h. During this period, the transfection efficiency was also assessed on the grounds of fluorescent reporter expression.

### 7. Isolation of lentiviruses

In order to isolate the virus, the culture medium was collected and centrifuged (3000 rpm, 10 min, 4°C) to remove dead cell remnants. Then the medium containing viruses was filtered (a 0.45-µm filter, washed with PBS) in preparation for ultracentrifugation. The cellular medium was centrifuged for 3h (50000G, 4°C) or 18h (20000G, 4°C). After the centrifugation, the supernatant was removed and the viral pellet was suspended in sterile PBS (1:300 of the initial medium volume). Lentiviruses obtained by this method were separated into 1.5-ml tubes at a volume of 20-50 µl per addition and stored at -80°C. Biological transduction efficiency was determined in HEK293 cells and viruses (and their concentrations), which produced more than 40% GFP-positive infected cells were intended for further use.

### 8. Generation of iNS cells from fibroblasts/keratinocytes using a prepared lentivirus

Fibroblastic cells and keratinocytes collected from patients were infected with obtained retroviruses. A transduction of each of the cell lines with hSox2 and c-Myc coding viruses was performed. The collected cells were maintained in a MEM medium supplemented with 10% fetal bovine serum. After achieving a 40-80% confluence, these cells were infected. After viral transduction (48h), the culture medium was changed to a medium supporting reprogramming to iNS cells and a selection of cells with a medium supplemented with blasticidin (5 µg/ml) was performed. After a one-week's selection, a morphological assessment of the cells and immunohistochemical analysis of specific marker expression were carried out. During subsequent three weeks, colonies of cells reprogrammed to iNS cells were transferred on a layer of fibroblasts treated with mitomycin (feeder layer) in a ESC (embryonic stem cells) medium. About one month later a differentiation of iNS cells into glial and neuronal cells was performed.

### Example 2.2

### Protocol for generation of iNS cells using the transcription factors Sox2 and c-Myc and an adenoviral vector

The steps 1-4 are identical with the steps in Example 2.1:
1. Isolation of cellular RNA.
2. Reverse transcription reaction and obtainment of cDNA.
3. Amplification of Sox 2 and c-Myc coding sequences.
4. Cloning of transcription factors into the pIRESneo3 vector.
5. Mutagenesis of the pShuttle-CMV-LacZ vector.

A site-specific mutagenesis of a shuttle vector intended for cloning of an insert containing a transcription factor was performed to facilitate further DNA procedures. A modification of the Ndel restriction site (cutting site 7745) in the pShuttle-CMV-LacZ vector by exchanging two nucleotides was performed using the following primers:

| | |
|---|---|
| pShuttle mut Fwd | 5'- AAT TAA CAT GCA TGG ATC CAT GCG CGG TGT GAA ATA CCG CAC -3'; |
| pShuttle mut Rev | 5'-GTG CGG TAT TTC ACA CCG CGC ATG GAT CCA TGC ATG TTA ATT -3'. |

Primer elongation reaction was performed using a Pfull Ultra HS repair activity DNA polymerase (Stratagene) according to the following mutagenesis programme:

| | | | | | |
|---|---|---|---|---|---|
| 1. | Denaturation | 95°C | 1 min | | |
| 2. | Denaturation | 95°C | 50 s | | |
| | Annealing | 60°C | 50 s | } | 18 cycles |
| | Elongation | 68°C | 11 min | | |
| 3. | Final elongation | 68°C | 7 min | | |

The reaction product was digested with Dpnl restriction enzyme that acts on bacterial methylated plasmid DNA, which makes it possible to collect the digestion product without removing the non-methylated mutagenesis product. After purification (AxyPrep PCR Cleanup, AxyGen), the mutant plasmid was transformed and propagated in *E. coli* cells. The mutagenesis reaction correctness was confirmed by an analysis using Ndel restriction enzyme and electrophoretic analysis of digested DNA.

### 6. Subcloning of the CMV-TF¹-IRES-tGFP(2A)Blast-WPRE fragment into the pShuttle vector

2 µg of pShuttle-CMV-LacZ(mut) plasmid were digested with Ndel/Blpl restriction enzymes and then dephosphorylated with antarctic phosphatase (NEB). 2 µg of pLOC-hSox2 lentiviral plasmid and of pLOC-c- Myc lentiviral plasmid that contain the CMV-TF-tGFP(2A)Blast-WPRE fragment were digested with NdeI/Bsu36I enzymes. Afterwards, a separation of DNA in agarose gel was performed and bands corresponding with the vector (∼7.3 kbp) and insert (∼4.3 and 4.7 kbp) were isolated and cleared of the gel (AxyPrep Gel extraction, AxyGen). Ligation reaction of insert to shuttle vector was performed with T4 DNA ligase (NEB) at 12°C for 16 h. 50 ng of vector, 200 ng of insert and 0.5 µl of enzyme were used for the reaction. The ligation product was introduced into chemically competent *E. coli* cells. An expansion of a number of isolated bacterial colonies in a LB medium with ampicillin and then an isolation of DNA and digestion with Xhol/Notl restriction enzymes to confirm the presence of a complete insert (expected: 2 bands - 10 kbp and 1.6/1.9 kbp).

### 7. Homologous recombination in vivo between the pShuttle vector and adenoviral vector pAdEasy-1

In order to obtain a complete adenoviral vector, a homologous recombination between pShuttle-hSox2 (and pShuttle-c-Myc) shuttle plasmid and E1-defective vector pAdEasy-1 containing the other part of the adenoviral genome was performed. The electrocompetent *E. coli* strain BJ5183-AD-1 (pretransformed with the Stratagene plasmid pAdEasy-1) that contains the native recA gene was used for the recombination. The pShuttle-hSox2 plasmid was linearized with Pmel restriction enzyme and then ∼100 ng of it were added to the bacterial suspension. The electroporation parameters were: 200 Ω, 2.5 kV, 25 µF. After an ∼1h incubation in a SOC medium at 37°C, the cells were
¹TF - transcription factor transferred into a selective agar medium with kanamycin. The following day, 10 bacterial colonies were selected and after an ∼8h incubation and expansion in a LB medium with kanamycin DNA was isolated (AxyPrep Plasmid Miniprep, Axygen). Recombinant adenoviral vectors were identified by restriction analysis using Pacl enzyme (expected: 2 products - ∼32 kbp and 3 kbp).

The DNA obtained from positively identified colonies was then used for a transformation of recA mutant and end1-negative chemically competent *E. coli* cells (Turbo, NEB). Afterwards, a plasmid containing recombinant complete adenoviral genome was isolated and purified (using AxyPrep Plasmid Maxiprep, Axygen) from these *E. coli* cells.

### 8. Transfection of AD-293 packaging cells and generation of initial adenoviral plaques

Dishes intended for packaging cell cultures were coated with poly-L-lysine (5 ml of 0.0005% solution for ∼60 min). AD-293 cells were transferred into a DMEM culture medium at a volume of 6x10⁶ cells per addition to a dish and their uniform distribution was ensured. These cells were maintained at 37°C in an atmosphere of 5% CO₂. The next day, the cells were washed with PBS and 10 ml of fresh culture medium were added. The transfection of AD-293 packaging cells was performed using PEI (Polyethylenimine, Polysciences). DNA of a complete adenoviral vector linearized with Pacl restriction enzyme containing the hSox2 sequence and was used for the transfection. Transfection in one 10-cm-diameter dish was carried out according to the following scheme: 20µl of PEI (1µ g/µl) were added to 680 µl of 150 mM NaCl in a 1.5-ml tube. 5µg of a linearized vector were placed in a tube and the contents were filled up to the final volume of 700 µl with 150 mM NaCl. Then, a PEI solution in NaCl was added to the tube containing DNA, the contents were mixed, centrifuged and left for a 30 minute incubation at room temperature. Afterwards, the solution containing PEI/DNA complexes was transferred drop by drop into the AD-293 cell culture medium. The dish was placed in an incubator for 12 h. The following day, the culture medium was removed and the cells were gently coated with 3.5ml of 1% low-melting agarose (heated to 42°C) in a culture medium. When the agarose has solidified, 7 ml of DMEM (with glucose and antibiotics) were added to each dish. The cells were maintained in an incubator at 37°C in an atmosphere of 5% CO₂. After 5 days, the liquid medium was removed and the next layer consisting of 3.5 ml of 1% agarose was placed in DMEM. Adenoviral plaques should be observable 7-10 days after transfection.

### 9. Initial adenoviral plaque expansion in AD-293 packaging cells

Ten days after transfection, the adenoviral plaques were aspirated using pipettes with sterile 1-ml tips (with a filter to avoid equipment contamination). The plaques were suspended in 0.5 ml of PBS and four cycles of freezing-defreezing (-80°C-37°C) were performed to release the adenovirus. In order to amplify the adenovirus, 500 µl of a suspension containing primary viruses were added to a T150 culture flask medium containing AD-293 packaging cells (at ∼70% confluence). The infected cells were maintained in an incubator at 37°C in an atmosphere of 5% CO₂ for 5-7 days till the appearance of observable cell lysis. The collected culture medium (containing cells) underwent four cycles of freezing-defreezing (-80°C - 37°C) to release the adenovirus and then it was centrifuged (at 3000 rpm, 4°C, 5 min) to separate cell remnants. The harvested supernatant (crude extract) was used for isolation of adenoviruses.

### 10. Isolation and purification of adenoviruses

Adenoviruses were isolated using caesium chloride gradient ultracentrifugation. 2.5 ml of a CsCI solution (δ=1.4 g/ml) in TBS were placed in a 50-ml ultracentrifugation tube. The upper layer was formed by a CsCI solution with a density δ=1.25 g/ml. 5 ml of crude adenoviral extract were added to the CsCI gradient and then it was coated with 2 mm of mineral oil to avoid aerosolization and apparatus contamination with the virus. The samples prepared by this method were centrifuged (35000 rpm, 1h, 15°C). After centrifugation, the white band at the CsCI gradient phase boundary was collected using a disposable syringe with a 20G needle (the lower and upper band was formed by proteins of an empty adenoviral capsid). The isolated adenovirus was dialyzed in a 4% glucose solution in PBS and the obtained product was then portioned 20-50 µl by 20-50 µl and stored at -80°C. Biological transduction efficiency was determined in HEK293T cells and viruses (and their concentrations), which produced at least 50% GFP-positive infected cells were intended for further use.

### 11. Generation of iNS cells from fibroblasts/keratinocytes with a prepared adenovirus

Like the lentiviral vector, fibroblastic cells and keratinocytes collected from patients were infected with the obtained adenoviruses. Each of the cell lines was transduced with hSox2 coding virus. The collected cells were maintained in a MEM supplemented with 10% fetal bovine serum. After achieving a 40-80% confluence, these cells were infected. After viral transduction (48h), the culture medium was changed to a medium supporting reprogramming to iNS cells. A selection of cells was performed using a medium supplemented with blasticidin (5µg/ml). After a one-week's selection, a morphological assessment of the cells and immunohistochemical analysis of specific marker expression was carried out. During subsequent three weeks, colonies of cells reprogrammed to iNS cells were transferred on a layer of fibroblasts treated with mitomycin (feeder layer) in a ESC (embryonic stem cells) medium. One month later, a differentiation of iNS cells into glial and neuronal cells was performed.

### Example 2.3

### Protocol for generation of iNS cells using the transcritpion factors Sox2 and c-Myc and a retroviral vector

The steps 1-4 are identical with the steps in Example 2.1:
1. Isolation of cellular RNA.
2. Reverse transcription reaction and obtainment of cDNA.
3. Amplification of Sox 2 and c-Myc coding sequences.
4. Cloning of transcription factors into the pIRESneo3 vector.
5. Subcloning of the CMV-TF-IRES-tGFP(2A)Blast-WPRE fragment into the pFB retroviral vector.

2 µg of pFB plasmid were digested with EcoRI/Notl restriction enzymes, incubated with mung bean nuclease and dephosphorylated with antarctic phosphatase (NEB). 2 µg of pLOC-hSox2 and pLOC-c-Myc lentiviral plasmid containing the hSox2-tGFP(2A)Blast-WPRE and c-Myc-tGFP(2A)Blast-WPRE fragment were digested with ClaI/Bsu36I enzymes, incubated with mung bean nuclease to obtain blunt ends and a phosphorylated with T4 polynucleotide kinase. The DNA was separated in agarose gel and bands corresponding with the vector (∼5.3 kbp) and insert (∼3.7/4.1 kbp) were isolated and cleared of the gel (AxyPrep Gel extraction, AxyGen). The ligation reaction of a shuttle vector and insert containing the Sox2 (or c-Myc) sequence was performed with T4 DNA ligase (NEB) at 12°C for 16 h. 50ng of vector, 200 ng of insert and 0.5 µl of enzyme were used for the reaction. The ligation product was introduced into chemically competent *E. coli* cells. An expansion of a number of isolated bacterial colonies in LB medium with ampicillin and then DNA isolation and digestion with Bsu36I/BamHI restriction enzymes to confirm the presence of a correct insert orientation (expected: 2 bands - 7.2 kbp and 1.7/2.1 kbp) were performed.

### 6. Transfection of HEK293T packaging cells with the pFB vector

The dishes for maintaining packaging cells were coated with poly-L-lysine (5 ml of 0.0005% solution for ∼60 min). The HEK293T cells were introduced into a DMEM culture medium at a volume of 6x10⁶ cells per addition to a dish and their uniform distribution was ensured. The cells were maintained at 37°C in an atmosphere of 5% CO₂. On the next day, the cells were washed with PBS and 10 ml of fresh culture medium were added. The transfection of HEK293T packaging cells was performed using PEI (Polyethylenimine, Polysciences). The transfection of one 10cm diameter dish was performed as follows: 40 µL of PEI (1 µg/µl) were added to 660 µl of 150 mM NaCl in a 1.5 ml tube. The 3 µg of pFB vector coding for proper transcription factor were mixed with 3 µg of pVPack-GP and 3 µg of plasmids coding for envelope proteins (pVPack-Ampho or pVPack-10A1) in another tube. The mixture was filled up to the final volume of 700µl with 150 mM NaCl. Then, the NaCl solution with PEI was transferred to the tube containing DNA. The tube contents were mixed, centrifuged and incubated at room temperature for 30 min. Then, the solution containing PEI/DNA complexes was transferred drop by drop into the medium with cultured HEK 293T cells. The dish was placed in an incubator for 8-12 h. On the next day, the culture medium was removed and replaced with a DMEM solution without serum (with added glucose and antibiotics). The cells were maintained under this conditions for 40-48 h. During this period, the transfection efficiency was assessed on the grounds of fluorescent reporter expression.

### 7. Isolation of retroviruses

In order to isolate the viruses, the culture medium was centrifuged (3000 rpm, 10 min, 4°C) to remove dead cell remnants. Then, the medium containing retroviruses was filtered (a 0.45-µm filter, washed with PBS) in preparation for ultracentrifugation. The cellular medium was centrifuged for 3 h (50000G, 4°C) or 18 h (20000G, 4°C). After the centrifugation, the supernatant was removed and a viral pellet was suspended in sterile PBS (1:300 of the initial medium volume). The obtained retroviruses were placed in 1.5-ml tubes with screw caps (to avoid environment contamination with the retroviruses) (20-50 µl in each tube) and stored at -80°C. The biological transduction efficiency in HEK293 cells was determined and only the viruses (and their concentrations), which produced more than 40% GFP-positive infected cells, were used in further procedures.

### 8. Generation of iNS cells from fibroblasts/keratinocytes using a prepared retrovirus

Fibroblastic cells and keratinocytes collected from patients were infected with the obtained retroviruses. Each of these cell lines were transduced at the same time with viruses coding for Sox2 and c-Myc factors. The collected cells were maintained in a MEM medium supplemented with 10% fetal bovine serum. After achieving a 40-80% confluence, these cells were infected. Following the transduction with the virus (48h), the culture medium was changed to a medium supporting reprogramming to iNS cells and a selection of the cells was carried out using a medium with added blasticidin (5µg/ml). After a one-week's selection, a morphological assessment of the cells and an immunohistochemical analysis of specific marker expression were carried out.

### Example 2.4

### Protocol for generation of iNS cells using the transcritpion factors Sox2 and c-Myc and the piggyBac transposon vector

The steps 1-4 are identical with the steps in Example 2.1:
1. Isolation of cellular RNA.
2. Reverse transcription reaction and obtainment of DNA.
3. Amplification of Sox 2 and c-Myc coding sequences.
4. Cloning of transcription factors into the pIRESneo3 vector.
5. Subcloning of transcription factors into the CMV-TF-IRES-tGFP(2A)Blast-WPRE intermediate vector.
6. Subcloning of the CMV-TF-IRES-tGFP(2A)Blast-WPRE fragment into the site located between the inverted terminal repeats of a transposon.

2 µg of pPB51x-EF1α-Puro plasmid (SBI) were digested with Ascl/Fsel restriction enzymes (located outside the HS4 insulator sequence), incubated with mung bean nuclease and dephosphorylated with antarctic phosphatase (NEB). 2 µg of pLOC-hSox2 and pLOC-c-Myc lentiviral plasmid were digested with SanDI/Bsu36I enzymes, incubated with mung bean nuclease to obtain blunt ends and phosphorylated with T4 polynucleotide kinase. The DNA was separated in agarose gel and bands corresponding with the vector (∼5.5 kbp) and the insert (^{∼}4.5 i 4.8 kbp) were isolated and cleared of the gel (AxyPrep Gel extraction, AxyGen). Ligation reaction of piggyBac vector and insert containing transcription factor was performed with T4 DNA ligase (NEB) under the control of CMV promoter at 12°C for 16 h. The reaction was performed with 50 ng of vector, 200 ng of insert and 0.5 µl of enzyme. The ligation product was introduced into chemically competent E. coli cells. An expansion of a number of isolated bacterial colonies was performed in a LB medium with ampicillin and then a DNA isolation and restriction analysis were carried out.

### 7. Transfection of primary fibroblastic cells/keratinocytes with a prepared piggyBac vector and transposase coding plasmid

Cultured fibroblastic cells and keratinocytes from patients underwent transfection with prepared transposons and a plasmid coding for the enzymatic component - transposase (Super PiggyBac transposase plasmid (SBI) coding for optimized enzyme variant). To avoid an effect of reaction inhibition caused by defective enzyme variants (overproduction inhibition), the DNA was introduced at 1:5 ratio (transposase/transposon) that is optimal for this system. The transfection was performed with the FuGENE® HD (Roche) reagent. 9 µg of DNA (1.5 µg of piggyBac transposase coding plasmid and 7.5 µg of transposon) were transferred into 700 µl of DMEM (without antibiotics and serum) culture medium. Then, 18 µl of the FuGENE® reagent were added and the medium was filled up to the volume of 800 µl, mixed and left at room temperature for 15 min. Fibroblasts and keratinocytes were maintained in 10-cm-diameter dishes. The transfection was performed with cells located above the layer with 50% confluence. The FuGENE®/DNA mixture was transferred drop by drop into a cell culture medium, which was placed in an incubator for 48 h.

### 8. Selection of cells with a chromosomally integrated transposon and generation of iNS cells

The genomic integration of transposons was confirmed with PCR using primers specific for Sox2 and c-Myc. Two after the transfection, the cellular medium was removed and replaced with a medium supporting reprogramming to iNS cells. At the same time, a selection of cells using blasticidin (5 µg/mL) was started. After a selection lasting one week, a morphological assessment of the cells with an immunohistochemical analysis of specific marker expression were performed.

### 9. Transposon remobilization

The integrated transposon was removed through a transfection of reprogrammed cells with a plasmid coding only for transposase. A transfection was performed with 10 µg of Super PiggyBac transposase plasmid (SBI) in a 10-cm-diameter dish and the contents were transferred into 700 µl of DMEM culture medium (without added antibiotics and serum). Then, 20 µl of FuGENE® transfection reagent were added and the medium was filled up to the volume of 800 µl, mixed and left at room temperature for 15 min. Afterwards, the FuGENE®/DNA mixture was transferred drop by drop into the medium with cultured cells and without blasticidin, which was placed in an incubator for 48 h. The transposon remobilization (loss) was assessed on the grounds of reprogrammed cell fluorescence decay (caused by a tGFP(2A)Blast cassette). A morphological assessment of the cells obtained by this method and immunohistochemical analysis of specific marker expression were carried out. During subsequent three weeks, colonies of cells reprogrammed to iNS cells were transferred on a layer of fibroblasts treated with mitomycin (feeder layer) in a ESC medium (embryonic stem cells). About one month later, a differentiation of iNS cells into glial and neuronal cells was performed.

### Differentiation of iNS and iPS cells under standard conditions for obtainment of myocardial cells and hepatic cells

One of the undisputable advantages of cells obtained according to the described method is this, that no teratomas, i.e. neoplasms derived from pluripotent embryonal cells, originate due to their differentiation. In teratomas takes place a growth and differentiation of these cells into cell lines that have features of all germ layers, i.e. the ectoderm, endoderm, mesoderm, and sporadically of a trophoblast. Since iNS cells are not regressed to the level of cells that have such a versatile differentiation spectrum, they are not able to generate cells from a number of different germ layers.

In order to prove the qualities stated in the above description as usable ones in practice, experiments were carried out in which iPS and iNS cells obtained in a laboratory were differentiated into myocardial cells and hepatic cells simultaneously.

The iPS cells were obtained from fibroblasts using a CytoTune™ kit (Cat. No. A13780-01, Life Technologies). The iNS cells were obtained using a technique that was stated in the patent disclosure.

### Procedure for cell differentiation into hepatic cells

The iNS cells and iPS cells were cultured in the 1640 Medium (Hyclone, Logan, UT) with added 0.5 mg/mL albumin fraction V (Sigma-Aldrich) and 100 ng/mL activin A for 1 day simultaneously. During the next two days, the dose in the medium was gradually increased with 0.1% and 1% insulin-transferrin-selenium (ITS) (Sigma-Aldrich). On the third day after starting the supplementations with activin A, the medium was changed to a hepatocyte culture medium (HCM) (Cambrex, Baltimore, MD) enriched with 30 ng/mL FGF4 and 20 ng/mL BMP-2 and the culture was continued for 5 days. During the subsequent five-day's step, HCM dodano 20 ng/mL of HGF, 10 ng/mL oncostatin M and 0.1 µM dexamethasone were added to the medium. The medium was changed every day. Cells that had a positive staining for antibodies against human α-fetoprotein (AFP) and albumin (DAKO, Glostrup, Denmark) were obtained using this biotechnological method for iPS cells. An increased percent of GFAP-positive cells (a reaction with AFP or albumin was missing) was observed in treated with this method iNS cell cultures. This is an evidence of a considerably higher elasticity in the differentiation into iPS cells in comparison with iNS cells and cells produced by the process that have features of hepatocytes occur only in culture vessels, which initially contained iPS cells. Neural cells with a predominance of astrocytes (GFAP+) originated from iNS cells in culture vessels.

### Procedure for cell differentiation into myocardial cells

The iNS and iPS cells were cultured in DMEM without glutamine with a high glucose concentration and with added mitotically inactivated mouse embryonic fibroblasts (MEFs). The culture medium was enriched with 1 mmol/L L-glutamine, 0.1 mmol/L mercaptoethanol, 4 ng/mL recombinant human fibroblast growth factor (FGF) and 1% amino acids solution (Invitrogen, Carlsbad, Calif). Then, to induce differentiation, the cells were digested with collagenase type IV (1 mg/ml, 20 min, Life Technologies, Carlsbad, Calif) and small cell colonies were obtained, then transferred to plastic Petri dishes where they created embryoid bodies (EBs) and were cultured suspended for up to 10 days. The EBs were then inoculated onto culture vessels coated with 0.1% gelatin and cultured till achieving spontaneous cell contractions.
Spontaneous contractions were only observed in some percent of cells obtained by iPS cell differentiation. Such an activity was not shown by any cell obtained through iNS cell differentiation. These cells were GFAP-, MAP-2- and beta-tubulin-positive.

An additional advantage of using only reprogramming to iNS cells is this, that only cells from one neural line after the differentiation process are obtained. As regards cells obtained by differentiation of iPS cells, despite the use of many differentiation factors, apart from the desired cells, several different cells, relatively often completely different from the assumed list of the germ layers are obtained.

## Claims

1. A method for reprogramming somatic cells into induced neural stem (iNS) cells, wherein only two reprogramming factors comprising complete sequences that encode Sox2 and c-Myc protein are used, wherein somatic cells are fibroblasts or keratinocytes and the method produces iNs cells **characterized by** the presence of the markers Sox2, nestin (neural stem cell markers), and GFAP (glial fibrillary acidic protein).

2. The method according to claims 1, wherein the average reprogramming efficiency with the two reprogramming factors, i.e. Sox2 and c-Myc, equals to 0.1%.

3. The method according to claims 1 or 2, wherein the Sox2, GFAP, and Nestin positive cells show the ability to differentiate into cells that are **characterized by** the presence of MAP2+ and beta III+ markers in Neurobasal Medium (NBM) and DMEM/F-12 culture supplemented with bFGF differentiation factor.

## Patentansprüche

1. Eine Methode der Reprogrammierung somatischer Zellen in induzierte neurale Stammzellen (iNS), wobei nur zwei Reprogrammierungsfaktoren verwendet werden, die vollständige Sequenzen umfassen, die das Sox2- und das c-Myc-Protein kodieren, wobei somatische Zellen Fibroblasten oder Keratinozyten sind und das Verfahren iNS-Zellen erzeugt, die durch die Anwesenheit der Marker Sox2, Nestin (Neurale Stammzellmarker) und GFAP (Gliales Fibrilläres Saures Protein) gekennzeichnet sind.

2. Eine Methode nach Anspruch 1, wobei die durchschnittliche Reprogrammierungseffizienz mit den zwei Reprogrammierungsfaktoren, d. H. Sox2 und c-Myc, gleich 0,1% ist.

3. Verfahren nach den Ansprüchen 1 oder 2, wobei die Sox2-, GFAP- und Nestinpositiven Zellen die Fähigkeit zeigen, sich in Zellen zu differenzieren, die durch die Anwesenheit von MAP2 + und Beta III + Markern in Neurobasal Medium (NBM) und DMEM / F-12-Zellkultur ergänzt mit bFGF-Differenzierungsfaktor, gekennzeichnet sind.

## Revendications

1. Une méthode de reprogrammation des cellules somatiques dans les cellules souches neurales induites (iNS), où seulement deux facteurs de reprogrammation comprenant des séquences complètes qui codent Sox2 et la protéine c-Myc sont utilisés, où les cellules somatiques sont des fibroblastes ou des kératinocytes et la méthode produit des cellules iNS **caractérisées par** la présence des marqueurs Sox2, nestin (marqueurs de cellules souches neurales) et GFAP (protéine acide fibrillaire gliale).

2. Le procédé selon la revendication de brevet 1, où l'efficacité moyenne de reprogrammation avec les deux facteurs de reprogrammation, c'est à dire Sox2 et c-Myc, est égale à 0,1%.

3. Le procédé selon les revendications 1 ou 2, où les cellules positives Sox2, GFAP et Nestin montrent la capacité à se différencier dans cellules qui sont **caractérisées par** la présence de marqueurs MAP2 + et beta III + dans un milieu NeuroblasaI Medium (NBM) et une culture cellulaire DMEM / F-12 supplémenté avec un facteur de différenciation bFGF.
